# EUROPEAN PATENT APPLICATION

(11) **EP 3 604 293 A1**
(43) Date of publication of application: **05.02.2020**
(21) Application number: 18772518.9
(22) Date of filing: 22.03.2018
(51) Int. Cl.: C07D 317/38, H01M 10/0525, H01M 10/0569

(54) **METHOD FOR STABILIZING HIGH-PURITY ETHYLENE CARBONATE-CONTAINING COMPOSITION**

(30) Priority: 22.03.2017 JP 2017056662; 22.03.2017 JP 2017056663; 22.03.2017 JP 2017056664
(71) Applicant: Mitsubishi Chemical Corporation, Tokyo 100-8251 (JP)
(72) Inventor: MINEMOTO, Kouki, Tokyo 100-8251 (JP); ISOGAI, Takayuki, Tokyo 100-8251 (JP)
(74) Representative: Ter Meer Steinmeister & Partner
(86) International application number: PCT/JP2018/011520
(87) International publication number: WO 2018/174196

(57) **Abstract**

To provide a novel stabilization method for suppressing over-time denaturation of a high-purity ethylene carbonate-containing composition, a stabilized high-purity ethylene carbonate-containing composition, and the like. A method for stabilizing a high-purity ethylene carbonate-containing composition includes adjustment of content of the total of formic acid and a formic acid salt, or 2-chloroethanol to 500 ppm by mass or less in the high-purity ethylene carbonate-containing composition including 90% by mass or more ethylene carbonate.

## Description

### TECHNICAL FIELD

The present invention relates to a method for stabilizing a high-purity ethylene carbonate-containing composition, and a stabilized high-purity ethylene carbonate-containing composition, a method for storing the composition, an electrolytic solution for a lithium-ion battery, and the like.

### BACKGROUND ART

Ethylene carbonate is widely used in solvents for various polymer compounds, reaction solvents for various chemical reactions, extraction solvents, foaming agents, lubrication stabilizers, surfactants, soil modifiers, and the like. In recent years, use of ethylene carbonate has been increasingly expanded also in the fields of resist agents, resist stripping agents, pharmaceutical products, and the like. In particular, high-purity ethylene carbonate are industrially useful organic compounds as raw materials of electrolytic solutions for lithium-ion secondary batteries because of their high dielectric constant characteristics. Ethylene carbonate can also be hydrolyzed to selectively produce ethylene glycols useful for raw materials of polyesters, antifreeze solutions, and the like.

Methods for producing ethylene carbonate are known to be able to produce ethylene carbonate by a reaction of ethylene oxide and carbon dioxide. In the case where such a reaction is performed, products after the reaction include, in addition to ethylene carbonate as a main product, a catalyst used in the reaction and reaction by-products such as glycols including monoethylene glycol and diethylene glycol, and further include water and a hydrolysis catalyst in the case where hydrolysis is performed. In order to remove such impurities described above, ethylene carbonate is purified by a reduced-pressure distillation method, a cooling crystallization method, or the like (see, for example, Patent Literature 1 to 3).

### PRIOR ART DOCUMENTS

### Patent Literature

Patent Literature 1: JP H07-89905 A
Patent Literature 2: JP 2014-51484 A
Patent Literature 3: WO 2007/108213 A1

### SUMMARY OF THE INVENTION

### Technical Problem

In the case of use of ethylene carbonate as an electrolytic solution for a lithium-ion battery over a long period of time, such ethylene carbonate is denatured by an electrochemical reaction to cause a precipitate on the surface of an electrode and/or generation of a decomposed gas, thus degradation of a lithium-ion battery proceeds. Such degradation of a lithium-ion battery is considered to be caused specifically by the following phenomena. The first cause is an irreversible exchange reaction of a solvent due to the change in the composition of such an electrolytic solution, the second cause is precipitation of a reaction product and the like on the surface of an electrode due to increase of a resistant component, and the third cause is increase in inner pressure of the battery according to generation of any gas of hydrocarbon or the like. Such degradation can also be promoted due to the change in the composition of the electrolytic solution and the changes in viscosity, density and the like caused by the change in the composition, and the like.

It has been recently increasingly demanded to provide a high-purity ethylene carbonate, for example, one having a purity of 90% by mass or more. Meanwhile, there has not been heretofore focused on any specific behavior of minor components contained in a high-purity ethylene carbonate at all. However, it has been found according to studies by the present inventors that a high-purity ethylene carbonate not used (ethylene carbonate before use thereof as the electrolytic solution for a lithium-ion battery, in the above example) is undesirably denatured and/or changed in the composition over time (hereinafter, sometimes simply referred as "denaturation".).

Therefore, an urgent challenge is that the denaturation mechanism of an ethylene carbonate not used is clarified and such denaturation is suppressed, not only from the viewpoint that a high-quality ethylene carbonate is simply provided, but also from the viewpoint that high quality is maintained for a long period in use for various applications.

The present invention has been made in view of the above problems, and an object of a first embodiment and a second embodiment thereof is to provide a novel stabilization method for suppressing over-time denaturation of a high-purity ethylene carbonate-containing composition. Another object of the first embodiment and the second embodiment is to provide a stabilized high-purity ethylene carbonate-containing composition with suppressed over-time denaturation and a method for storing the composition, as well as an electrolytic solution for a lithium-ion battery, using the same, and the like. An object of a third embodiment is to provide a stabilized high-purity ethylene carbonate-containing composition, a method for storing the composition, an electrolytic solution for a lithium-ion battery, using the same, and the like. Another object of the third embodiment of the invention is to provide a production method which enables such a stabilized high-purity ethylene carbonate-containing composition to be stably produced.

There is not herein limited to the objects here mentioned, and exertion of any effects to be derived from each constitution indicated in modes for carrying out the invention, described below, the effects being not obtained by any conventional arts, can also be regarded as other objects of the present invention.

### Solution to Problem

The present inventors have made intensive studies in order to solve the above problems, and as a result, have found that the above problems can be solved by clarification of the denaturation mechanism in a high-purity ethylene carbonate-containing composition and adjustment of the composition to a predetermined composition, thus the present invention has been achieved.

That is, the first embodiment of the present invention provides various specific aspects indicated below.
[A1] A method for stabilizing a high-purity ethylene carbonate-containing composition, wherein the method comprises a step of adjustment of content (content rate) of the total of formic acid and a formic acid salt to 500 ppm by mass or less in the high-purity ethylene carbonate-containing composition comprising 90% by mass or more ethylene carbonate.
[A2] The method for stabilizing a high-purity ethylene carbonate-containing composition according to [A1], wherein the method further comprises a step of adjustment of content of iron oxide to less than 100 ppm by mass in the high-purity ethylene carbonate-containing composition.
[A3] The method for stabilizing a high-purity ethylene carbonate-containing composition according to [A1] or [A2], wherein the method further comprises a step of adjustment of content of water oxide to less than 100 ppm by mass in the high-purity ethylene carbonate-containing composition.
[A4] The method for stabilizing a high-purity ethylene carbonate-containing composition according to any one of [A1] to [A3], wherein the content of the ethylene carbonate in the high-purity ethylene carbonate-containing composition is 99.90% by mass or more.
[A5] A stabilized high-purity ethylene carbonate-containing composition comprising 90% by mass or more ethylene carbonate and 500 ppm by mass or less in total of formic acid and a formic acid salt.
[A6] The stabilized high-purity ethylene carbonate-containing composition according to [A5], wherein the content of the total of the formic acid and the formic acid salt is 100 ppm by mass or less.
[A7] The stabilized high-purity ethylene carbonate-containing composition according to [A5] or [A6], wherein the content of the total of the formic acid and the formic acid salt is 50 ppm by mass or less.
[A8] The stabilized high-purity ethylene carbonate-containing composition according to any one of [A5] to [A7], wherein the content of the total of the formic acid and the formic acid salt is 10 ppm by mass or less.
[A9] The stabilized high-purity ethylene carbonate-containing composition according to any one of [A5] to [A8], further comprising iron oxide, wherein the content of the iron oxide is less than 100 ppm by mass.
[A10] The stabilized high-purity ethylene carbonate-containing composition according to any one of [A5] to [A9], further comprising water, wherein the content of the water is less than 100 ppm by mass.
[A11] A method for storing a high-purity ethylene carbonate-containing composition, wherein the method comprises storing the high-purity ethylene carbonate-containing composition according to any one of [A5] to [A10], in a closed vessel or a sealed vessel.
[A12] An electrolytic solution for a lithium-ion battery, wherein the electrolytic solution comprises the high-purity ethylene carbonate-containing composition according to any one of [A5] to [A10].

The step of adjustment in each of [A1] to [A4] preferably comprises a step of measuring content of the total of formic acid and the formic acid salt in the high-purity ethylene carbonate-containing composition, and a step of selecting the high-purity ethylene carbonate-containing composition having the content of 500 ppm by mass or less.

That is, the second embodiment of the present invention provides various specific aspects indicated below.
[B1] A method for stabilizing a high-purity ethylene carbonate-containing composition, wherein the method comprises a step of adjustment of content (content rate) of 2-chloroethanol to 500 ppm by mass or less in the high-purity ethylene carbonate-containing composition comprising 90% by mass or more ethylene carbonate.
[B2] The method for stabilizing a high-purity ethylene carbonate-containing composition according to [B1], wherein the method further comprises a step of adjustment of content of iron oxide to less than 200 ppm by mass in the high-purity ethylene carbonate-containing composition.
[B3] The method for stabilizing a high-purity ethylene carbonate-containing composition according to [B1] or [B2], wherein the method further comprises a step of adjustment of content of water to less than 200 ppm by mass in the high-purity ethylene carbonate-containing composition.
[B4] The method for stabilizing a high-purity ethylene carbonate-containing composition according to any one of [B1] to [B3], wherein the content of the ethylene carbonate in the high-purity ethylene carbonate-containing composition is 99.90% by mass or more.
[B5] A stabilized high-purity ethylene carbonate-containing composition comprising 90% by mass or more ethylene carbonate and 500 ppm by mass or less of 2-chloroethanol.
[B6] The stabilized high-purity ethylene carbonate-containing composition according to [B5], wherein the content of the 2-chloroethanol is 100 ppm by mass or less.
[B7] The stabilized high-purity ethylene carbonate-containing composition according to [B5] or [B6], wherein the content of the 2-chloroethanol is 50 ppm by mass or less.
[B8] The stabilized high-purity ethylene carbonate-containing composition according to any one of [B5] to [B7], wherein the content of the 2-chloroethanol is 10 ppm by mass or less.
[B9] The stabilized high-purity ethylene carbonate-containing composition according to any one of [B5] to [B8], further comprising iron oxide, wherein the content of the iron oxide is less than 200 ppm by mass.
[B10] The stabilized high-purity ethylene carbonate-containing composition according to any one of [B5] to [9], further comprising water, wherein the content of the water is less than 200 ppm by mass.
[B11] A method for storing a high-purity ethylene carbonate-containing composition, wherein the method comprises storing the high-purity ethylene carbonate-containing composition according to any one of [B5] to [B10], in a closed vessel or a sealed vessel.
[B12] An electrolytic solution for a lithium-ion battery, wherein the electrolytic solution comprises the high-purity ethylene carbonate-containing composition according to any one of [B5] to [B10].

The step of adjustment in each of [B1] to [B4] also preferably comprises a step of measuring content of 2-chloroethanol in the high-purity ethylene carbonate-containing composition, and a step of selecting the high-purity ethylene carbonate-containing composition having the content of 500 ppm by mass or less.

That is, the third embodiment of the present invention provides various specific aspects indicated below.
[C1] A stabilized high-purity ethylene carbonate-containing composition comprising 90% by mass or more ethylene carbonate, and monoethylene glycol and/or monoethylene glycol formic acid ester, wherein the total content (total content rate) of the monoethylene glycol and the monoethylene glycol formic acid ester after a lapse of 125 days at 50°C in a closed vessel or a sealed vessel is 3.0% by area or less in gas chromatographic analysis.
[C2] A stabilized high-purity ethylene carbonate-containing composition comprising 90% by mass or more ethylene carbonate, and monoethylene glycol and/or monoethylene glycol formic acid ester, wherein the total content of the monoethylene glycol and the monoethylene glycol formic acid ester after a lapse of 100 days at 50°C in a closed vessel or a sealed vessel is 2.0% by area or less in gas chromatographic analysis.
[C3] A stabilized high-purity ethylene carbonate-containing composition comprising 90% by mass or more ethylene carbonate, and monoethylene glycol and/or monoethylene glycol formic acid ester, wherein the total content of the monoethylene glycol and the monoethylene glycol formic acid ester after a lapse of 50 days at 50°C in a closed vessel or a sealed vessel is 1.0% by area or less in gas chromatographic analysis.
[C4] The stabilized high-purity ethylene carbonate-containing composition according to any one of [C1] to [C3], further comprising formic acid and a formic acid salt, wherein the content of the total of the formic acid and the formic acid salt is 500 ppm by mass or less.
[C5] The stabilized high-purity ethylene carbonate-containing composition according to any one of [C1] to [C4], further comprising 2-chloroethanol, wherein the content of the 2-chloroethanol is 500 ppm by mass or less.
[C6] The stabilized high-purity ethylene carbonate-containing composition according to any one of [C1] to [C5], further comprising iron oxide, wherein the content of the iron oxide is less than 200 ppm by mass.
[C7] The stabilized high-purity ethylene carbonate-containing composition according to any one of [C1] to [C6], further comprising water, wherein the content of the water is less than 200 ppm by mass.
[C8] The stabilized high-purity ethylene carbonate-containing composition according to any one of [C1] to [C7], wherein the ethylene carbonate is ethylene carbonate which is obtained by carbonation method and which has a concentration of 2-chloroethanol of less than 0.1 ppm by mass and a content of formic acid and a formic acid salt of 0.1 ppm by mass.
[C9] A method for storing a high-purity ethylene carbonate-containing composition, wherein the method comprises storing the high-purity ethylene carbonate-containing composition according to any one of [C1] to [C8], in a closed vessel or a sealed vessel.
[C10] An electrolytic solution for a lithium-ion battery, wherein the electrolytic solution comprises the high-purity ethylene carbonate-containing composition according to any one of [C1] to [C8].
[C11] A method for producing a high-purity ethylene carbonate-containing composition, wherein the method comprises a reaction step of providing ethylene carbonate by reacting ethylene oxide and carbon dioxide, and a step of adjustment of the concentration of 2-chloroethanol and the content of formic acid and a formic acid salt to less than 0.1 ppm by mass in the resulting ethylene carbonate.
[C12] The method for producing a high-purity ethylene carbonate-containing composition according to [C11], wherein the method comprises providing a stabilized high-purity ethylene carbonate-containing composition comprising 90% by mass or more ethylene carbonate, and monoethylene glycol and/or monoethylene glycol formic acid ester, in which the total content of the monoethylene glycol and the monoethylene glycol formic acid ester is 3.0% by area or less in gas chromatographic analysis after a lapse of 125 days at 50°C in a closed vessel or a sealed vessel.
[C13] The method for producing a high-purity ethylene carbonate-containing composition according to [C11] or [C12], wherein the method comprises providing a stabilized high-purity ethylene carbonate-containing composition comprising 90% by mass or more ethylene carbonate, and monoethylene glycol and/or monoethylene glycol formic acid ester, in which the total content of the monoethylene glycol and the monoethylene glycol formic acid ester is 2.0% by area or less in gas chromatographic analysis after a lapse of 100 days at 50°C in a closed vessel or a sealed vessel.
[C14] The method for producing a high-purity ethylene carbonate-containing composition according to any one of [C11] to [C13], wherein the method comprises providing a stabilized high-purity ethylene carbonate-containing composition comprising 90% by mass or more ethylene carbonate, and monoethylene glycol and/or monoethylene glycol formic acid ester, in which the total content of the monoethylene glycol and the monoethylene glycol formic acid ester is 1.0% by area or less in gas chromatographic analysis after a lapse of 50 days at 50°C in a closed vessel or a sealed vessel.

The methods according to [C11] to [C14] each preferably provide a high-purity ethylene carbonate-containing composition having any one or more of [C4] to [C8], preferably a plurality of such features.

[D] A method for producing a high-purity ethylene carbonate-containing composition comprising 90% by mass or more ethylene carbonate, wherein the method comprises the following steps:
(1) A reaction step of providing an ethylene carbonate-containing composition by reacting ethylene oxide with carbon dioxide;
(2) A step of measuring content of the total (total content rate) of formic acid and a formic acid salt, or content (content rate) of 2-chloroethanol in the ethylene carbonate-containing composition; and
(3) A step of selecting the high-purity ethylene carbonate-containing composition having the content of the total of formic acid and the formic acid salt or the content of the 2-chloroethanol, of 500 ppm by mass or less.

### Advantageous Effects of Invention

According to the first embodiment or the second embodiment of the present invention, a novel stabilization method for suppressing over-time denaturation of a high-purity ethylene carbonate-containing composition can be realized. According to the first embodiment or the second embodiment of the present invention, a stabilized high-purity ethylene carbonate-containing composition with suppressed over-time denaturation and a method for storing the composition, as well as an electrolytic solution for a lithium-ion battery, using the same, and the like can also be realized.

According to the third embodiment of the present invention, a stabilized high-purity ethylene carbonate-containing composition with suppressed over-time denaturation and a method for storing the composition, as well as an electrolytic solution for a lithium-ion battery, using the same, and the like can be realized. A production method which enables such a stabilized high-purity ethylene carbonate-containing composition to be stably produced can also be realized.

### BRIEF DESCRIPTION OF DRAWINGS

Figure 1 is a graph illustrating the analysis results of Experimental Examples 1 to 2.
Figure 2 is a graph illustrating the analysis results of Experimental Examples 3 to 5.
Figure 3 is a graph illustrating the analysis results of Experimental Examples 6 to 9.
Figure 4 is a graph illustrating the analysis results of Experimental Examples 10 to 11.

### DESCRIPTION OF EMBODIMENTS

Hereinafter, embodiments of the present invention will be described in detail. The following embodiments are examples (representative examples) of aspects of the present invention, and the present invention is not intended to be limited thereto. The present invention can be arbitrarily modified and carried out without departing from the gist thereof. In the case of expression by use of "to" sandwiched between numerical values or physical property values described before and after such "to", the numerical values or physical property values used are assumed to be encompassed. For example, the expression of a numerical value range of "1 to 100" is intended to encompass both a lower limit value "1" and an upper limit value "100". Much the same is true on the expression of other numerical value range. The "monoethylene glycol" may be designated as "ethylene glycol" and the "monoethylene glycol formic acid ester" may be designated as "ethylene glycol formic acid ester" herein.

### [Method for stabilizing high-purity ethylene carbonate-containing composition, and stabilized high-purity ethylene carbonate-containing composition]

A method for stabilizing a high-purity ethylene carbonate-containing composition of a first embodiment includes adjustment of the content of the total of formic acid and a formic acid salt to 500 ppm by mass or less in a high-purity ethylene carbonate-containing composition comprising 90% by mass or more ethylene carbonate. A stabilized high-purity ethylene carbonate-containing composition of the present embodiment comprises 90% by mass or more ethylene carbonate and 500 ppm by mass or less in total of formic acid and a formic acid salt.

A method for stabilizing a high-purity ethylene carbonate-containing composition of a second embodiment includes adjustment of the content of 2-chloroethanol to 500 ppm by mass or less in a high-purity ethylene carbonate-containing composition comprising 90% by mass or more ethylene carbonate. A stabilized high-purity ethylene carbonate-containing composition of the present embodiment comprises 90% by mass or more ethylene carbonate and 500 ppm by mass or less of 2-chloroethanol.

A stabilized high-purity ethylene carbonate-containing composition of a third embodiment comprises 90% by mass or more ethylene carbonate, and monoethylene glycol and/or monoethylene glycol formic acid ester, in which the total content of the monoethylene glycol and the monoethylene glycol formic acid ester after a lapse of 125 days at 50°C in a closed vessel or a sealed vessel is 3.0% by area or less in gas chromatographic analysis.

In other words, the stabilized high-purity ethylene carbonate-containing composition of the present embodiment comprises 90% by mass or more ethylene carbonate, and monoethylene glycol and/or monoethylene glycol formic acid ester, in which the total content of the monoethylene glycol and the monoethylene glycol formic acid ester after a lapse of 100 days at 50°C in a closed vessel or a sealed vessel is 2.0% by area or less in gas chromatographic analysis.

In still other words, the stabilized high-purity ethylene carbonate-containing composition of the present embodiment comprises 90% by mass or more ethylene carbonate, and monoethylene glycol and/or monoethylene glycol formic acid ester, in which the total content of the monoethylene glycol and the monoethylene glycol formic acid ester after a lapse of 50 days at 50°C in a closed vessel or a sealed vessel is 1.0% by area or less in gas chromatographic analysis.

As described above, a method including reacting ethylene oxide with carbon dioxide is known as a method for producing ethylene carbonate, and is progressively put in practical use. Formic acid and 2-chloroethanol are known to be able to be included, as impurities, in ethylene oxide in a step of generating such ethylene oxide, and such formic acid as impurities included in ethylene oxide can be incorporated into a high-purity ethylene carbonate-containing composition. However, there has not been heretofore focused on any specific behavior caused by formic acid and/or 2-chloroethanol contained in a high-purity ethylene carbonate-containing composition, at all.

On the contrary, the present inventors have found that formic acid and a formic acid salt contained in a high-purity ethylene carbonate-containing composition unexpectedly each serve as a trigger substance which increases monoethylene glycol (MEG), formic acid esters such as ethylene glycol monoformic acid ester, or the like (sometimes collectively referred them to as "by-product impurities".) over time and increase in such by-product impurities causes over-time deterioration in quality of the high-purity ethylene carbonate-containing composition. The present inventors simultaneously newly have found that there are acceptable amounts of formic acid and a formic acid salt in the high-purity ethylene carbonate-containing composition from the viewpoint that the composition is maintained at a high quality for a predetermined period.

The present inventors have also found that 2-chloroethanol contained in a high-purity ethylene carbonate-containing composition unexpectedly serves as a trigger substance which increases by-product impurities including monoethylene glycol (MEG), formic acid esters such as ethylene glycol monoformic acid ester, or the like over time and increase in such by-product impurities causes over-time deterioration in quality of the high-purity ethylene carbonate-containing composition. The present inventors simultaneously newly have found that there is an acceptable amount of 2-chloroethanol in the high-purity ethylene carbonate-containing composition from the viewpoint that the composition is maintained at a high quality for a predetermined period.

The present inventors further have also found that formic acid and a formic acid salt, and 2-chloroethanol contained in a high-purity ethylene carbonate-containing composition unexpectedly each serve as a trigger substance which increases by-product impurities including monoethylene glycol (MEG), formic acid esters such as ethylene glycol monoformic acid ester, or the like over time and increase in such by-product impurities causes over-time deterioration in quality of the high-purity ethylene carbonate-containing composition. The present inventors simultaneously newly have found that there are acceptable increase rates with respect to such increases of by-product impurities in the high-purity ethylene carbonate-containing composition from the viewpoint that the composition is maintained at a high quality for a predetermined period.

Hereinafter, the present invention will be described in more detail.

### (Ethylene carbonate)

The content of ethylene carbonate in the high-purity ethylene carbonate-containing composition of the first embodiment and the second embodiment is not particularly limited, and the content based on the total amount of the high-purity ethylene carbonate-containing composition is usually 90.00% by mass or more, preferably 95.00% by mass or more, more preferably 99.00% by mass or more, further preferably 99.50% by mass or more, particularly preferably 99.90% by mass or more, most preferably 99.95% by mass or more, further most preferably 99.99% by mass or more. The content of the ethylene carbonate is equal to or more than such a preferable lower limit value, then a destabilization phenomenon due to other impurities is relatively decreased and the stabilization effect of the present invention tends to be relatively actualized.

While the ethylene carbonate here used is not particularly limited and a new synthetic product or a commercially available product can be used without any distinction, any ethylene carbonate (hereinafter, sometimes simply referred as "ethylene carbonate obtained by carbonation method".) is preferable which is obtained through a reaction step of providing an ethylene carbonate-containing composition by reacting ethylene oxide with carbon dioxide (hereinafter, sometimes simply referred as "carbonation reaction step".) and a purification step of purifying the ethylene carbonate-containing composition. An ethylene carbonate obtained by carbonation method, obtained by such a production method, can include formic acid and/or 2-chloroethanol serving as impurities in relatively large amount(s), and thus the stabilization effect of the present invention tends to be relatively actualized in the case of use of such an ethylene carbonate. It is further preferable to obtain an ethylene carbonate-containing composition by use of a carbonation catalyst described below, in the carbonation reaction.

Ethylene oxide is industrially produced by, usually, allowing ethylene and oxygen to react in the presence of a silver catalyst. The rate of selection of ethylene to ethylene oxide is usually about 80%, and the residue, about 20% of such ethylene, is completely oxidized and transformed into carbon dioxide and water. Ethylene oxide for use as a raw material of ethylene carbonate to be industrially produced usually includes about 40% of water. Alternatively, such ethylene oxide may include diols such as monoethylene glycol and diethylene glycol due to partial hydrolysis of ethylene carbonate.

The purity of the ethylene oxide for use as a raw material is not particularly limited, and is usually 50% by mass or more, preferably 55% by mass or more, more preferably 60% by mass or more. The upper limit is not particularly limited, and is preferably 95% by mass or less, more preferably 90% by mass or less, further preferably 85% by mass or less from the viewpoint of economic efficiency, availability, and the like.

The molar ratio of raw materials fed in the carbonation reaction step (carbon dioxide/ethylene oxide) is not particularly limited, and is usually 0.5 or more as a rough standard. The upper limit of the molar ratio thereof fed is also not particularly limited, and is usually 5 or less, preferably 3.0 or less.

The carbonation reaction step of the ethylene oxide is usually performed in the presence of a catalyst. Such a carbonation catalyst may be appropriately selected from known catalysts, and used, and the type is not particularly limited. Specific examples include alkali metal halide; alkali earth metal halide; alkylamine; a quaternary ammonium salt; organic tin; germanium; a tellurium compound; a halogenated organic phosphonium salt, and alkali metal carbonate, but are not particularly limited thereto. Among them, alkali metal halide and a halogenated organic phosphonium salt are preferable. Alkali metal carbonate is preferable because generation of by-products can be easily suppressed.

Specific examples of the alkali metal halide include alkali metal iodides such as potassium iodide and alkali metal bromides such as potassium bromide. The alkali metal here is preferably potassium high in solubility. Specific examples of the alkali earth metal halide include alkali earth metal iodide and alkali earth metal bromide.

Specific examples of the halogenated organic phosphonium salt include quaternary phosphonium iodides such as triphenylmethylphosphonium iodide, triphenylpropylphosphonium iodide, triphenylbenzylphosphonium iodide and tributylmethylphosphonium iodide, and quaternary phosphonium bromides such as triphenylmethylphosphonium bromide, triphenylpropylphosphonium bromide, triphenylbenzylphosphonium bromide and tributylmethylphosphonium bromide. Specific examples of the alkali metal carbonate include potassium carbonate.

The amount of the catalyst used in the carbonation reaction step is not particularly limited, and is preferably 1/1000 or more, more preferably 1/200 or more in terms of the molar ratio thereof to the ethylene oxide. The upper limit is not particularly limited, and is preferably 1/20 or less, more preferably 1/50 or less. The carbonation catalyst can be used singly or in any combination of two or more kinds at any ratio. The amount thereof in the case of use of a plurality of the carbonation catalysts preferably falls within the above range as the total amount.

The temperature at which the carbonation reaction step of the ethylene oxide is performed is not particularly limited, and is usually 70°C or more, preferably 100°C or more. On the other hand, the upper limit is usually 200°C or less, preferably 170°C or less. The reaction pressure is also not particularly limited, and is usually 0.6 MPa or more, preferably 1.0 MPa or more. On the other hand, the upper limit is usually 5.0 MPa or less, preferably 3.0 MPa or less.

The carbonation reaction step of the ethylene oxide can be performed using any apparatus. The carbonation reaction step can be performed in either a batch manner or a continuous feeding manner, and is preferably performed in a continuous feeding manner in terms of industry. Specifically, for example, the reaction can be performed by control of the reaction temperature by circulating a reaction liquid in a tower through a liquid circulation conduit using a bubble tower having a heat exchanger and the liquid circulation conduit provided with a circulation pump, and by continuous supply of ethylene oxide and carbon dioxide which are raw materials and a catalyst from the tower bottom. A reactor provided with an ejector type nozzle is also preferably used. In the case where the reaction is performed by use of the bubble tower, a tube reactor is preferably disposed following the bubble tower to allow unreacted ethylene oxide to further react in terms of reaction efficiency.

The ethylene carbonate obtained by carbonation method, obtained by such a production method, can include formic acid and a formic acid salt, 2-chloroethanol, and the like as impurities in relatively large amounts. Such formic acid and formic acid salt, and 2-chloroethanol each serve as a trigger substance which causes by-product impurities such as monoethylene glycol and formic acid ester of monoethylene glycol to be increased over time, and increase in such by-product impurities causes over-time deterioration in quality of the high-purity ethylene carbonate-containing composition. Therefore, a step of reducing the contents of 2-chloroethanol and a formic acid and a formic acid salt in the ethylene carbonate obtained by carbonation method is preferably further performed. The contents of 2-chloroethanol and a formic acid and a formic acid salt in the ethylene carbonate obtained by carbonation method are not particularly limited, and are each desirably adjusted to less than 0.1 ppm by mass. The contents of 2-chloroethanol and a formic acid and a formic acid salt in the ethylene carbonate obtained by carbonation method are more preferably lower, and preferably 0 ppm in view of effects of the present invention. However, for example, a measurement apparatus for detecting formic acid and a formic acid salt is limited in terms of technique (detection limit, lower limit of quantitation), and thus the contents of 2-chloroethanol and a formic acid and a formic acid salt in the ethylene carbonate obtained by carbonation method is preferably less than the detection limit of such a measurement apparatus.

Such a reduction in contents of 2-chloroethanol, and formic acid and a formic acid salt can be performed according to an ordinary method, and the method therefor is not particularly limited. Examples of the method for such a reduction include any separation method by recrystallization, crystallization, extraction by suspending and washing, concentration, washing with water, dewatering, filtration, distillation, distillation off, rectification, chromatography, a cationic exchange resin, or an alcohol adsorbent. Such a reduction in contents of 2-chloroethanol, and formic acid and a formic acid salt may also be performed in a purification step described below.

The purification step is to purify the ethylene carbonate obtained in the carbonation reaction step. The purification can be performed by a crystallization method, a distillation method, or the like. The purification is preferably performed by a crystallization method, more preferably a countercurrent contact type crystallization method, from the viewpoint of excellent energy efficiency. In the case where the purification is performed by a crystallization method, an ethylene carbonate crystal is usually produced by cooling a reaction liquid. For example, a wall surface falling type crystallization method obtains a high-purity ethylene carbonate by precipitating a crystal on a wall surface cooled, thereafter warming the crystal and melting it, and allowing it to flow down. A countercurrent contact type crystallization method obtains a high-purity ethylene carbonate by generating a crude crystal of ethylene carbonate by cooling or the like of a reaction liquid, and countercurrently contacting a crystal melt liquid obtained by heating the crude crystal, with such a crude crystal not molten.

The crude crystal of ethylene carbonate can be precipitated by a method including adding a poor solvent to the reaction liquid subjected to the carbonation reaction, a method including cooling the reaction liquid subjected to the carbonation reaction, or the like. It is preferable in terms of industry to precipitate the crude crystal of ethylene carbonate by cooling of the reaction liquid. The crude crystal of ethylene carbonate can be taken out by solid-liquid separation of a liquid including the unreacted ethylene oxide and the catalyst.

It is specifically preferable in the countercurrent contact type crystallization method in terms of industry to allow the crude crystal of ethylene carbonate to fall from the upper portion of the tower, melt the crude crystal of ethylene carbonate at the bottom of the tower, extract a part of the resulting melt liquid from the tower, and allow the balance of the melt liquid to rise as a reflux liquid and countercurrently contact it with the falling crude crystal of ethylene carbonate.

In the case where a hydrolysis reaction of ethylene carbonate is performed, such a hydrolysis reaction is preferably performed in the presence of a known hydrolysis catalyst such as an alkali metal compound. Specific examples of the hydrolysis catalyst include compounds of alkali metals such as sodium and potassium. The alkali metal contained in such an alkali metal compound is preferably potassium. Examples of the alkali metal compound include alkali metal hydroxide; and alkali metal carbonate and bicarbonate. Among them, the alkali metal compound is preferable and alkali metal carbonate is further preferable as the hydrolysis catalyst. In the case where the carbonation reaction and the hydrolysis reaction are simultaneously performed, the hydrolysis catalyst here used is preferably present at a molar ratio thereof to the carbonation catalyst, of 0.01 to 1.0. The hydrolysis catalyst can be used singly or in any combination of two or more kinds at any ratio. The amount thereof use in the case of a plurality of the hydrolysis catalysts preferably falls within the above range as the total amount.

### (Formic acid or Formic acid salt)

In the first embodiment, formic acid serving as the trigger substance may be present in the form of a salt. Examples of the salt of formic acid serving as the trigger substance include metal salts such as an alkali metal salt or an alkali earth metal salt.

The content of the formic acid and the formic acid salt in the high-purity ethylene carbonate-containing composition of the present embodiment is 500 ppm by mass or less in total, preferably 100 ppm by mass or less in total, more preferably 50 ppm by mass or less in total, further preferably 10 ppm by mass or less in total based on the total amount of the high-purity ethylene carbonate-containing composition. On the other hand, the lower limit value of the total content of the formic acid and the formic acid salt is not particularly limited, and is 0 ppm as a rough standard in view of the effects of the present invention. In fact, however, a measurement apparatus for detecting the formic acid and the formic acid salt is limited in terms of technique (detection limit, detection ability), and thus an enhancement in analysis technique is required for supporting any behavior of the measurement apparatus at a value less than the detection limit. Thus, in the case where gas chromatograph is used in the measurement apparatus for detecting the formic acid and the formic acid salt, the lower limit is preferably set to a value equal to or more than the detection limit of the measurement apparatus, more preferably 0.1 ppm by mass or more in total, further preferably 0.5 ppm by mass in total, particularly preferably 1.0 ppm by mass or more in total.

The total content of the formic acid and the formic acid salt is equal to or less than the upper limit value, then increase in monoethylene glycol and formic acid ester of monoethylene glycol (such as monoethylene glycol monoformic acid ester) over time can be suppressed. The contents of the formic acid and the formic acid salt can be herein measured by gas chromatography. The procedure for adjusting the total content of the formic acid and the formic acid salt is not particularly limited, and can be performed by, for example, removal of the formic acid or the formic acid salt included in the high-purity ethylene carbonate-containing composition, addition of the formic acid or the formic acid salt to the high-purity ethylene carbonate-containing composition, or no shipping of one which contains the formic acid and the formic acid salt in amounts measured, out of the specification defined as the respective standard values of the amounts of the formic acid and the formic acid salt. Such an adjustment may include a step of measuring the total content of formic acid and the formic acid salt and a step of selecting one where the total content is equal to or less than a specific amount.

### (2-Chloroethanol)

The content of 2-chloroethanol in the high-purity ethylene carbonate-containing composition of the second embodiment is 500 ppm by mass or less, preferably 100 ppm by mass or less, more preferably 50 ppm by mass or less, further preferably 10 ppm by mass or less based on the total amount of the high-purity ethylene carbonate-containing composition. On the other hand, the lower limit value of the content of the 2-chloroethanol is not particularly limited, and is 0 ppm as a rough standard in view of the effects of the present invention. In fact, however, a measurement apparatus for detecting the 2-chloroethanol is limited in terms of technique (detection limit, detection ability), and thus an enhancement in analysis technique is required for supporting any behavior of the measurement apparatus at a value less than the detection limit. Thus, in the case where gas chromatograph is used in the measurement apparatus for detecting the 2-chloroethanol, the lower limit is preferably set to a value equal to or more than the detection limit of the measurement apparatus, more preferably 0.1 ppm by mass or more, further preferably 0.5 ppm by mass, particularly preferably 1.0 ppm by mass or more.

The content of the 2-chloroethanol is equal to or less than the upper limit value, then increase in monoethylene glycol and a formic acid ester of monoethylene glycol (such as monoethylene glycol monoformic acid ester) over time can be suppressed. The content of the 2-chloroethanol can be herein measured by gas chromatography. The adjustment of the content of the 2-chloroethanol is not particularly limited, and can be performed by, for example, removal of the 2-chloroethanol included in the high-purity ethylene carbonate-containing composition, addition of the 2-chloroethanol to the high-purity ethylene carbonate-containing composition, or no shipping of one which contains the 2-chloroethanol in an amount measured, out of the specification defined as the standard value of the amount of the 2-chloroethanol. Examples of removal of the trigger substance include any separation method by a cationic exchange resin, an alcohol adsorbent, or distillation. The adjustment may include a step of measuring the content of the 2-chloroethanol and a step of selecting one where the total content is equal to or less than a specific amount.

### (Monoethylene glycol and monoethylene glycol formic acid ester)

The contents of monoethylene glycol and monoethylene glycol formic acid ester in the high-purity ethylene carbonate-containing composition of the third embodiment are increased due to the trigger substance over time, and the amounts thereof to be increased are varied depending on the storage period and the storage temperature. Thus, in the present embodiment, the amounts are defined as the total content of the monoethylene glycol and the monoethylene glycol formic acid ester in predetermined conditions, namely, after a lapse of a certain period at 50°C in a closed vessel or a sealed vessel.

Specifically, the total content of the monoethylene glycol and the monoethylene glycol formic acid ester after a lapse of 125 days at 50°C in a closed vessel or a sealed vessel is 3.0% by area or less, preferably 2.5% by area or less, more preferably 2.0% by area or less, further preferably 1.75% by area or less, particularly preferably 1.5% by area or less in gas chromatographic analysis. The total content of the monoethylene glycol and the monoethylene glycol formic acid ester in the case of a lapse of 100 days is preferably 2.0% by area or less, more preferably 1.75% by area or less, further preferably 1.5% by area or less, particularly preferably 1.25% by area or less in gas chromatographic analysis. The total content of the monoethylene glycol and the monoethylene glycol formic acid ester in the case of a lapse of 50 days is preferably 1.0% by area or less, more preferably 0.75% by area or less, further preferably 0.5% by area or less, particularly preferably 0.25% by area or less in gas chromatographic analysis.

The total content of the monoethylene glycol and the monoethylene glycol formic acid ester after a lapse of a certain period at 50°C in a closed vessel or a sealed vessel is equal to or less than such a preferable upper limit value, then the quality reliability of the high-purity ethylene carbonate-containing composition which is exposed in various environments from production through distribution to use tends to be further ensured.

On the other hand, the lower limit value of the total content of the monoethylene glycol and the monoethylene glycol formic acid ester after a lapse of a certain period at 50°C in a closed vessel or a sealed vessel is not particularly limited, and is 0% by area as a rough standard in view of the effects of the present invention. In fact, gas chromatographic analysis is limited in terms of technique (detection limit, lower limit of quantitation), and thus an enhancement in analysis technique is required for supporting any behavior of a measurement apparatus at a value less than the detection limit. Thus, the lower limit is preferably set to a value equal to or more than the detection limit of a gas chromatographic analyzer, more preferably 0.001% by area or more, further preferably 0.01% by area or more, particularly preferably 0.1% by area or more.

The adjustment of the total content of the monoethylene glycol and the monoethylene glycol formic acid ester is not particularly limited, and can be performed by, for example, removal of the formic acid or the formic acid salt, or the 2-chloroethanol, water or iron oxide, included in the high-purity ethylene carbonate-containing composition, or addition thereof to the high-purity ethylene carbonate-containing composition. While the initiation in such lapsed days is usually defined as the point of time of production of high-purity ethylene carbonate, the lapsed days are not necessarily needed to be counted severely from the time of point of the production because of a tendency of increase in the contents of the monoethylene glycol and the monoethylene glycol formic acid ester over time, and the total content of the monoethylene glycol and the monoethylene glycol formic acid ester can be determined to be within the present embodiment as long as at least the total content after a predetermined lapsed days is equal to or less than the above predetermined value.

### (Other components)

The high-purity ethylene carbonate-containing compositions of the first embodiment to the third embodiment may each further include iron oxide in impurities. In such a case, the content of the iron oxide is preferably 200 ppm by mass or less, more preferably 150 ppm by mass or less, more preferably less than 100 ppm by mass, more preferably 50 ppm by mass or less, more preferably 25 ppm by mass or less, more preferably 10 ppm by mass or less, more preferably 5 ppm by mass or less based on the total amount of the high-purity ethylene carbonate-containing composition. The content of the iron oxide herein means a value obtained by measurement according to inductively coupled plasma optical emission spectroscopy (ICP/OES). The content of iron oxide in ethylene carbonate can be quantitatively determined by, for example, ICP/OES after decomposition of the ethylene carbonate by use of any acid such as nitric acid or sulfuric acid. The lower limit value of the content of the iron oxide is not particularly limited, and is 0 ppm as a rough standard in view of the effects of the present invention. In fact, however, the lower limit value is preferably equal to or more than the detection limit in the inductively coupled plasma optical emission spectroscopy, more preferably 0.1 ppm by mass or more, further preferably 0.5 ppm by mass, particularly preferably 1.0 ppm by mass or more for the reason of the presence of the detection limit of the measurement apparatus, as in the above formic acid and the formic acid salt, and the 2-chloroethanol.

The iron oxide of interest here encompasses three components of FeO, Fe₂O₃, and Fe₃O₄. In the case where both the formic acid or the formic acid salt and/or the 2-chloroethanol and the iron oxide are included, a remarkable increase in the monoethylene glycol and the formic acid ester thereof (for example, monoethylene glycol monoformic acid ester) over time may occur, but, not only the contents of the formic acid and the formic acid salt, and the 2-chloroethanol are adjusted as described above, but also the content of the iron oxide is adjusted to less than the above preferable upper limit value, then increase in the monoethylene glycol and the formic acid ester of monoethylene glycol over time tends to be stably suppressed. The adjustment of the content of the iron oxide is not particularly limited, and can be performed by, for example, removal of the iron oxide included in the high-purity ethylene carbonate-containing composition or addition of to the iron oxide to the high-purity ethylene carbonate-containing composition.

The high-purity ethylene carbonate-containing compositions of the first embodiment to the third embodiment may each include water in impurities. In such a case, the content of the water in the first embodiment is preferably less than 100 ppm by mass, more preferably 50 ppm by mass or less, further preferably 25 ppm by mass or less, particularly preferably 10 ppm by mass or less based on the total amount of the high-purity ethylene carbonate-containing composition. The content of the water in the second embodiment to the third embodiment is preferably less than 200 ppm by mass, more preferably 100 ppm by mass or less, further preferably 50 ppm by mass or less, particularly preferably 25 ppm by mass or less, most preferably 10 ppm by mass or less based on the total amount of the high-purity ethylene carbonate-containing composition. The content of the water herein means a value obtained by measurement with a Karl Fischer moisture titrator. The lower limit value of the content of the water is not particularly limited, and is 0 ppm as a rough standard in view of the effects of the present invention. In fact, however, the lower limit value is preferably equal to or more than the detection limit of the Karl Fischer moisture titrator, more preferably 0.1 ppm by mass or more, further preferably 0.5 ppm by mass, particularly preferably 1.0 ppm by mass or more for the reason of the presence of the detection limit of the measurement apparatus, as in the above formic acid or the formic acid salt, and the 2-chloroethanol and the iron oxide.

In the case where both the formic acid or the formic acid salt and/or the 2-chloroethanol and the water are included, a remarkable increase in the monoethylene glycol and the formic acid ester of monoethylene glycol (for example, monoethylene glycol monoformic acid ester) over time may occur, but, not only the contents of the formic acid and the formic acid salt, and the 2-chloroethanol are adjusted as described above, but also the content of the water is adjusted to less than the above preferable upper limit value, then increase in the monoethylene glycol and the formic acid ester of monoethylene glycol over time tends to be stably suppressed. The adjustment of the content of the water is not particularly limited, and can be performed by, for example, removal of the water included in the high-purity ethylene carbonate-containing composition or addition of to the water to the high-purity ethylene carbonate-containing composition.

The high-purity ethylene carbonate-containing compositions of the first embodiment to the third embodiment may be each prepared by mixing respective components, or prepared by appropriately separating and removing any component from respective mixed components so that the contents of the respective components fall within predetermined ranges. The method for separating any component is not particularly limited, and can be selected with respect to each of the components. Here, the contents of the formic acid and the formic acid salt, and the 2-chloroethanol may be adjusted to 500 ppm by mass or less, and, if necessary, the contents of the iron oxide and the water may be adjusted to less than 100 ppm by mass in the first embodiment, and to 200 ppm by mass or less in the second embodiment and the third embodiment.

The high-purity ethylene carbonate-containing composition of the first embodiment, thus obtained by adjustment of the contents of the formic acid and the formic acid salt, and, if necessary, the contents of the iron oxide and the water to predetermined rates is hardly increased in by-product impurities such as monoethylene glycol (MEG) and formic acid ester of MEG even after a lapse of time, is excellent in storage stability, and is high in purity and high in quality.

The high-purity ethylene carbonate-containing composition of the second embodiment, thus obtained by adjustment of the content of the 2-chloroethanol, and, if necessary, the contents of the iron oxide and the water to predetermined rates is hardly increased in by-product impurities such as monoethylene glycol (MEG) and formic acid ester of MEG even after a lapse of time, is excellent in storage stability, and is high in purity and high in quality.

The high-purity ethylene carbonate-containing composition of the third embodiment, having such a composition, is hardly increased in by-product impurities such as monoethylene glycol (MEG) and formic acid ester of MEG even after a lapse of time, is excellent in storage stability, and is high in purity and high in quality.

### [Method for storing high-purity ethylene carbonate-containing composition]

The ethylene carbonate-containing compositions of the first embodiment to the third embodiment are each preferably stored with being not contact with air and water, specifically, preferably stored in a closed vessel or a sealed vessel from the viewpoint of suppression of increase in by-product impurities due to incorporation of moisture in the air. The compositions are each more preferably stored under a low oxygen partial pressure atmosphere and/or a low steam partial pressure atmosphere, for example, a vacuum or inert gas (nitrogen, argon, helium) atmosphere.

The storage temperature is not particularly limited, and is preferably 10 to 70°C, more preferably 10 to 60°C, further preferably 10 to 55°C. The storage temperature falls within the above range, then the effect of suppression of increase in by-product impurities such as monoethylene glycol (MEG) and formic acid ester of MEG over time tends to be favorably exerted.

The closed vessel or the sealed vessel including each of the ethylene carbonate-containing compositions of the first embodiment to the third embodiment is also one of embodiments of the present invention.

### [Method for producing high-purity ethylene carbonate-containing composition]

The high-purity ethylene carbonate-containing compositions comprising 90% by mass or more ethylene carbonate of the first embodiment to the third embodiment can be each produced by a method including the following steps:
(1) A reaction step of providing an ethylene carbonate-containing composition by reacting ethylene oxide with carbon dioxide;
(2) A step of measuring content of the total of formic acid and a formic acid salt, or content of 2-chloroethanol in the ethylene carbonate-containing composition in the ethylene carbonate-containing composition; and
(3) A step of selecting the high-purity ethylene carbonate-containing composition having the content of the total of the formic acid and the formic acid salt or the content of the 2-chloroethanol, of 500 ppm by mass or less.

### [Method for managing quality of high-purity ethylene carbonate-containing composition]

The high-purity ethylene carbonate-containing compositions comprising 90% by mass or more ethylene carbonate of the first embodiment to the third embodiment can be each managed in quality by a method including the following steps:
(1) A step of providing an ethylene carbonate-containing composition by reacting ethylene oxide with carbon dioxide;
(2) A step of measuring content of the total of formic acid and a formic acid salt, or content of 2-chloroethanol in the ethylene carbonate-containing composition; and
(3) A step of selecting the high-purity ethylene carbonate-containing composition having the content of the total of the formic acid and the formic acid salt or the content of the 2-chloroethanol, of 500 ppm by mass or less.

### [Electrolytic solution for lithium-ion battery]

The electrolytic solutions for a lithium-ion battery of the first embodiment to the third embodiment each include the above high-purity ethylene carbonate-containing composition. The high-purity ethylene carbonate-containing composition of the present embodiment includes less by-product impurities such as monoethylene glycol (MEG) or formic acid ester of MEG and has suppressed increase in such by-product impurities over time, and thus use thereof in an electrolytic solution for a lithium-ion battery can allow undesirable degradation in performance of a lithium-ion battery, caused by such by-product impurities, to be suppressed, then the battery capacity and the battery performance of a lithium-ion battery can be maintained for a longer period and a lithium-ion battery having high reliability during use for a long period of time.

Lithium-ion batteries are expected to be demanded in applications of not only small batteries, but also large batteries, and furthermore industrial batteries, and the field thereof is expected to be increasingly grown. Therefore, it can be said that the high-purity ethylene carbonate-containing composition of the present embodiment, which can enhance reliability during use for a long period of time, is extremely useful as a solvent of an electrolytic solution also from the viewpoint that a need for a higher-performance lithium-ion battery is expected in future.

The electrolytic solution for a lithium-ion battery may include any component(s) known in the art, and the composition thereof is not particularly limited. For example, an electrolytic solution for a lithium-ion battery is commercially available, which includes a lithium salt such as LiPF₆ or LiBOB, a non-aqueous solvent such as saturated cyclic carbonate, linear carbonate, linear carboxylate, cyclic carboxylate, an ether-based compound or a sulfone-based compound, and various additives known in the art. In particular, the high-purity ethylene carbonate-containing composition is particularly preferably used as an additive or a non-aqueous solvent for an electrolytic solution for a lithium-ion battery.

### EXAMPLES

Hereinafter, the content of the present invention will be more specifically described with reference to Experimental Examples, but the present invention is not intended to be limited to such Experimental Examples at all. Herein, various values in production conditions or as evaluation results in Experimental Examples each have the meaning as a preferable value of an upper limit or a lower limit in embodiments of the present invention, and a preferable range may be any range defined by a combination of the value of the upper limit or the lower limit with a value in the following Experimental Examples, or a combination of values in such Experimental Examples.

### [Experimental Example 1]

First, carbon dioxide, ethylene oxide, water, tributylmethylphosphonium iodide, and potassium carbonate were continuously fed to a reactor, and the resulting reaction liquid was purified by a countercurrent contact type crystallization method and then subjected to solid-liquid separation to provide a high-purity ethylene carbonate having a purity of 99.999%, including formic acid and a formic acid salt, 2-chloroethanol, and iron oxide at each rate less than the detection limit, and about 5 ppm by mass of water.

Next, 0.01 parts by mass of formic acid (100 ppm by mass based on the total amount of the composition) was added to 100 parts by mass of the resulting high-purity ethylene carbonate to prepare a high-purity ethylene carbonate-containing composition of Experimental Example 1.

Thereafter, the resulting high-purity ethylene carbonate-containing composition was placed in a 20-mL glass vial and sealed, and further placed in an open drum and then stored in an oven SPH-100 set to 50°C, manufactured by Tabai ESPEC Corporation.

After a lapse of a predetermined period from the start of storing, 0.8 mL of the high-purity ethylene carbonate-containing composition was sampled from the glass vial, and 0.2 mL of acetonitrile was added to prepare each sample solution for gas chromatography. In the case where any solid content was found in the sampling, the sample was, if necessary, subjected to filtration by 0.2-µm PTFE disc filter. The sample solution prepared was used to perform gas chromatographic analysis in the following conditions, and the percentage by area of each peak derived from ethylene glycol and ethylene glycol monoformic acid ester was calculated.

### [Analysis conditions]

Apparatus: GC-2010 plus manufactured by Shimadzu Corporation
Inlet temperature: 130°C
Amount of injection: 1.0 µL
Column: Agilent DB-WAXetr 123-7364
Column temperature: kept at 60°C for 2 minutes, thereafter raised to 200°C at a rate of rise of 20°C/min and kept for 16 minutes, and thereafter raised to 230°C at a rate of rise of 20°C/min and kept for 5 minutes
Carrier gas: helium, flow rate 50 cm/sec
Injection method: splitless (1:20 split after one minute of injection)
Detector: hydrogen flame ionization detector (FID) at 250°C
Post-treatment: ejection at an injection temperature of 230°C and a column temperature of 230°C for 5 minutes with respect to one measurement
Washing solvent: acetonitrile
Area calculation method: area percentage method excluding any peak derived from acetonitrile
Detection limit: 7 ppm by mass

### [Experimental Example 2]

A high-purity ethylene carbonate-containing composition of Experimental Example 2 was prepared in the same manner as in Experimental Example 1 except that the amount of formic acid compounded was changed to 0.001 parts by mass (10 ppm by mass based on the total amount of the composition).

Gas chromatographic analysis was performed in the same manner as in Experimental Example 1 except that the high-purity ethylene carbonate-containing composition of Experimental Example 2 was used instead of the high-purity ethylene carbonate-containing composition of Experimental Example 1.

### [Experimental Example 3]

A high-purity ethylene carbonate-containing composition of Experimental Example 3 was prepared in the same manner as in Experimental Example 1 except that 0.0001 parts by mass of water (1 ppm by mass based on the total amount of the composition) was further added to 100 parts by mass of the high-purity ethylene carbonate.

Gas chromatographic analysis was performed in the same manner as in Experimental Example 1 except that the high-purity ethylene carbonate-containing composition of Experimental Example 3 was used instead of the high-purity ethylene carbonate-containing composition of Experimental Example 1.

### [Experimental Example 4]

A high-purity ethylene carbonate-containing composition of Experimental Example 4 was prepared in the same manner as in Experimental Example 3 except that the amount of formic acid compounded was changed to 0.001 parts by mass (10 ppm by mass based on the total amount of the composition).

Gas chromatographic analysis was performed in the same manner as in Experimental Example 1 except that the high-purity ethylene carbonate-containing composition of Experimental Example 4 was used instead of the high-purity ethylene carbonate-containing composition of Experimental Example 1.

### [Experimental Example 5]

A high-purity ethylene carbonate-containing composition of Experimental Example 5 was prepared in the same manner as in Experimental Example 3 except that the amount of formic acid compounded was changed to 0.0001 parts by mass (1 ppm by mass based on the total amount of the composition).

Gas chromatographic analysis was performed in the same manner as in Experimental Example 1 except that the high-purity ethylene carbonate-containing composition of Experimental Example 5 was used instead of the high-purity ethylene carbonate-containing composition of Experimental Example 1.

### [Experimental Example 6]

A high-purity ethylene carbonate-containing composition of Experimental Example 6 was prepared in the same manner as in Experimental Example 1 except that 0.01 parts by mass of 2-chloroethanol (100 ppm by mass based on the total amount of the composition) was added instead of formic acid.

Gas chromatographic analysis was performed in the same manner as in Experimental Example 1 except that the high-purity ethylene carbonate-containing composition of Experimental Example 6 was used instead of the high-purity ethylene carbonate-containing composition of Experimental Example 1.

### [Experimental Example 7]

A high-purity ethylene carbonate-containing composition of Experimental Example 7 was prepared in the same manner as in Experimental Example 6 except that 0.001 parts by mass of water (10 ppm by mass based on the total amount of the composition) and 0.01 parts by mass of iron oxide (Fe₃O₄) (100 ppm by mass based on the total amount of the composition) were further added to 100 parts by mass of the high-purity ethylene carbonate.

Gas chromatographic analysis was performed in the same manner as in Experimental Example 1 except that the high-purity ethylene carbonate-containing composition of Experimental Example 7 was used instead of the high-purity ethylene carbonate-containing composition of Experimental Example 1.

### [Experimental Example 8]

A high-purity ethylene carbonate-containing composition of Experimental Example 8 was prepared in the same manner as in Experimental Example 7 except that the amount of water compounded was changed to 0.01 parts by mass (100 ppm by mass based on the total amount of the composition).

Gas chromatographic analysis was performed in the same manner as in Experimental Example 1 except that the high-purity ethylene carbonate-containing composition of Experimental Example 8 was used instead of the high-purity ethylene carbonate-containing composition of Experimental Example 1.

### [Experimental Example 9]

A high-purity ethylene carbonate-containing composition of Experimental Example 9 was prepared in the same manner as in Experimental Example 7 except that the amount of 2-chloroethanol compounded was changed to 0.001 parts by mass (10 ppm by mass based on the total amount of the composition).

Gas chromatographic analysis was performed in the same manner as in Experimental Example 1 except that the high-purity ethylene carbonate-containing composition of Experimental Example 9 was used instead of the high-purity ethylene carbonate-containing composition of Experimental Example 1.

### [Experimental Example 10]

A high-purity ethylene carbonate-containing composition of Experimental Example 10 was prepared in the same manner as in Experimental Example 7 except that the amount of 2-chloroethanol compounded was changed to 0.1 parts by mass (1000 ppm by mass based on the total amount of the composition).

Gas chromatographic analysis was performed in the same manner as in Experimental Example 6 except that the high-purity ethylene carbonate-containing composition of Experimental Example 10 was used instead of the high-purity ethylene carbonate-containing composition of Experimental Example 1.

### [Experimental Example 11]

A high-purity ethylene carbonate-containing composition of Experimental Example 11 was prepared in the same manner as in Experimental Example 8 except that the amount of 2-chloroethanol compounded was changed to 0.1 parts by mass (1000 ppm by mass based on the total amount of the composition).

Gas chromatographic analysis was performed in the same manner as in Experimental Example 6 except that the high-purity ethylene carbonate-containing composition of Experimental Example 11 was used instead of the high-purity ethylene carbonate-containing composition of Experimental Example 1.

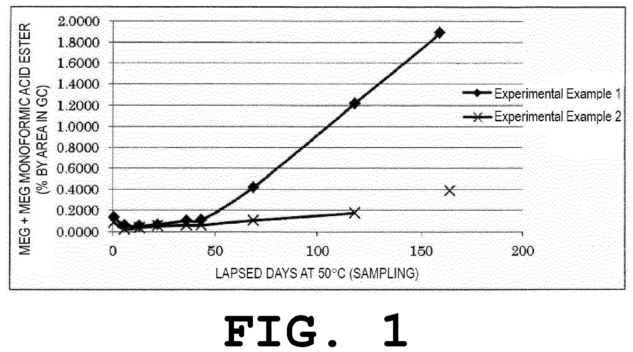

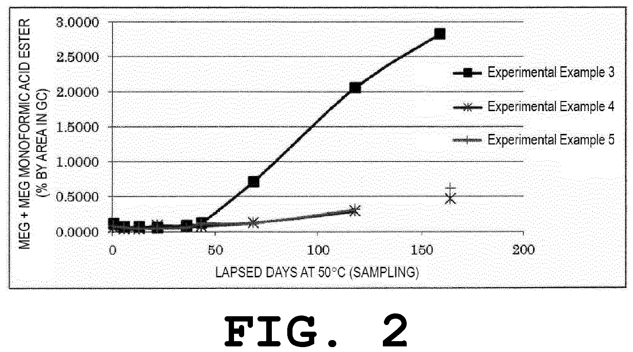

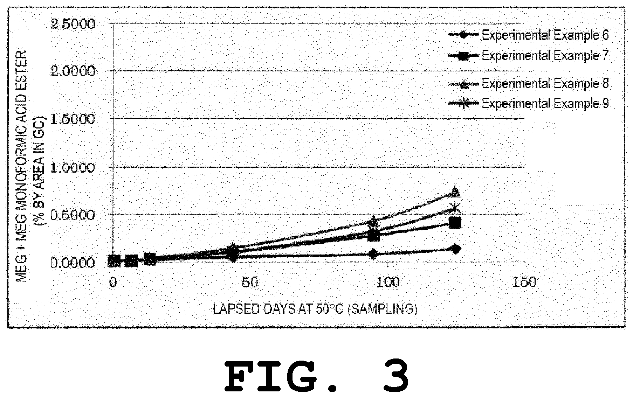

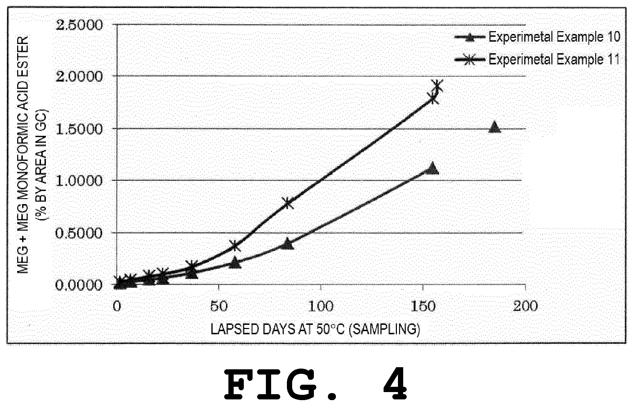

The results of each of Experimental Examples are shown in Tables 1 to 3 and illustrated in Figures 1 to 4.

Herein, the amounts of formic acid, 2-chloroethanol, water, and iron oxide in Tables 1 to 3 each represent the amount before storage of the high-purity ethylene carbonate of each of Experimental Examples.

**[Table 1]**

| | | Experimental Example 1 | Experimental Example 2 | Experimental Example 3 | Experimental Example 4 | Experimental Example 5 |
|---|---|---|---|---|---|---|
| Formic acid (ppm by mass) | | 100 | 10 | 100 | 10 | 1 |
| Water (ppm by mass) | | 0 | 0 | 1 | 1 | 1 |
| MEG and MEG monoformic acid ester (% by area) | 1 day | 0.136 | 0.0858 | 0.1096 | 0.0495 | 0.0769 |
| | 43 day | 0.1103 | 0.0617 | 0.1224 | 0.077 | 0.1077 |
| | 69 day | 0.4242 | 0.107 | 0.7129 | 0.1175 | 0.1188 |
| | 118 day | 1.2166 | 0.1786 | 2.0541 | 0.2832 | 0.307 |
| | 159 day | 1.8895 | | 2.8241 | | |
| | 164 day | | 0.3885 | | 0.4562 | 0.6102 |

| | | | | | | |
|---|---|---|---|---|---|---|
| MEG: ethylene glycol MEG monoformic acid ester: ethylene glycol monoformic acid | | | | | | |

**[Table 2]**

| | | Experimental Example 6 | Experimental Example 7 | Experimental Example 8 | Experimental Example 9 |
|---|---|---|---|---|---|
| 2-Chloroethanol (ppm by mass) | | 100 | 100 | 100 | 10 |
| Water (ppm by mass) | | 0 | 10 | 100 | 10 |
| Iron oxide (ppm by mass) | | 0 | 100 | 100 | 100 |
| MEG and MEG monoformic acid ester (% by area) | 1 day | 0.0136 | 0.0133 | 0.0132 | 0.0124 |
| | 44 day | 0.0529 | 0.1043 | 0.1466 | 0.1066 |
| | 95 day | 0.0819 | 0.2801 | 0.4347 | 0.3247 |
| | 125 day | 0.1374 | 0.4099 | 0.7344 | 0.5682 |

| | | | | | |
|---|---|---|---|---|---|
| MEG: ethylene glycol MEG monoformic acid ester: ethylene glycol monoformic acid | | | | | |

**[Table 3]**

| | | Experimental Example 10 | Experimental Example 11 |
|---|---|---|---|
| 2-Chloroethanol (ppm by mass) | | 1000 | 1000 |
| Water (ppm by mass) | | 10 | 100 |
| Iron oxide (ppm by mass) | | 100 | 100 |
| MEG and MEG monoformic acid ester (% by area) | 1 day | 0.0137 | 0.025 |
| | 37 day | 0.1138 | 0.1707 |
| | 58 day | 0.2139 | 0.3744 |
| | 84 day | 0.3991 | 0.7802 |
| | 155 day | 1.1256 | 1.7915 |
| | 185 day | 1.5201 | |

| | | | |
|---|---|---|---|
| MEG: ethylene glycol MEG monoformic acid ester: ethylene glycol monoformic acid | | | |

### INDUSTRIAL APPLICABILITY

The high-purity ethylene carbonate-containing composition, the stabilization method thereof, and the like of the present invention can be widely and effectively utilized in various applications such as solvents for various polymer compounds, reaction solvents for various chemical reactions, extraction solvents, foaming agents, lubrication stabilizers, surfactants, soil modifiers, resist agents, resist stripping agents, pharmaceutical products, raw materials of polyesters, antifreeze solutions, and the like, and among them, can be specially effectively utilized in an electrolytic solution for a lithium-ion battery.

## Claims

1. A method for stabilizing a high-purity ethylene carbonate-containing composition,
wherein the method comprises
a step of adjustment of content of the total of formic acid and a formic acid salt to 500 ppm by mass or less in the high-purity ethylene carbonate-containing composition comprising 90% by mass or more ethylene carbonate.

2. A method for stabilizing a high-purity ethylene carbonate-containing composition,
wherein the method comprises
a step of adjustment of content of 2-chloroethanol to 500 ppm by mass or less in the high-purity ethylene carbonate-containing composition comprising 90% by mass or more ethylene carbonate.

3. The method for stabilizing a high-purity ethylene carbonate-containing composition according to claim 1 or 2,
wherein the step of adjustment comprises
a step of measuring the content of the total of formic acid and the formic acid salt, or the content of 2-chloroethanol in the high-purity ethylene carbonate-containing composition, and
a step of selecting the high-purity ethylene carbonate-containing composition having the content of 500 ppm by mass or less.

4. The method for stabilizing a high-purity ethylene carbonate-containing composition according to any one of claims 1 to 3,
wherein the method further comprises
a step of adjustment of content of iron oxide to less than 100 ppm by mass in the high-purity ethylene carbonate-containing composition.

5. The method for stabilizing a high-purity ethylene carbonate-containing composition according to any one of claims 1 to 4,
wherein the content of the ethylene carbonate in the high-purity ethylene carbonate-containing composition is 99.90% by mass or more.

6. A stabilized high-purity ethylene carbonate-containing composition comprising
90% by mass or more ethylene carbonate, and
500 ppm by mass or less in total of formic acid and a formic acid salt.

7. A stabilized high-purity ethylene carbonate-containing composition,
wherein the composition comprises
90% by mass or more ethylene carbonate, and
500 ppm by mass or less of 2-chloroethanol.

8. A method for producing a high-purity ethylene carbonate-containing composition comprising 90% by mass or more ethylene carbonate,
wherein the method comprises the following steps:
(1) a reaction step of providing an ethylene carbonate-containing composition by reacting ethylene oxide with carbon dioxide;
(2) a step of measuring content of the total of formic acid and a formic acid salt, or content of 2-chloroethanol in the ethylene carbonate-containing composition; and
(3) a step of selecting the high-purity ethylene carbonate-containing composition having the content of the total of formic acid and the formic acid salt or the content of 2-chloroethanol, of 500 ppm by mass or less.

9. An electrolytic solution for a lithium-ion battery,
wherein the electrolytic solution comprises:
the high-purity ethylene carbonate-containing composition according to claim 6 or 7.
